# EUROPÄISCHE PATENTANMELDUNG

(11) **EP 1 815 791 A2**
(43) Veröffentlichungstag der Anmeldung: **08.08.2007**
(21) Anmeldenummer: 07001248.9
(22) Anmeldetag: 20.01.2007
(51) Int. Cl.: A61B 5/15

(54) **Stechgerät mit Impedanzmesseinrichtung**

(30) Priorität: 04.02.2006 EP 06002329
(71) Anmelder: Roche Diagnostics GmbH, 68305 Mannheim (DE); F. Hoffmann-Roche AG, CH-4070 Basel (CH)
(72) Erfinder: Grundmann, Armin, 72762 Reutlingen (DE)
(74) Vertreter: Mommer, Niels

(57) **Zusammenfassung**

Die Erfindung betrifft ein Stechgerät zum Erzeugen einer Einstichwunde zur Gewinnung einer Körperflüssigkeitsprobe eines Menschen oder Tieres, umfassend einen Stechelementantrieb (8), um ein in das Stechgerät (7) eingesetztes Stechelement (1) für eine Einstich- und Rückführbewegung anzutreiben, eine Wechselspannungsquelle (9), die dafür eingerichtet ist, ein Wechselspannungssignal an eine in ein eingesetztes Stechelement (1) integrierte Elektrode (6) anzulegen, eine elektrische Messeinrichtung (10), um ein durch das Anlegen des Wechselspannungssignals an die Elektrode (6) hervorgerufenes Antwortsignal zu messen, und eine Auswerteeinrichtung (13), die im Betrieb durch Auswertung des Antwortsignals ein Auswerteergebnis ermittelt und den Stechelementantrieb (8) unter Berücksichtigung des Auswerteergebnisses steuert. Erfindungsgemäß ist vorgesehen, dass die Wechselspannungsquelle (9) derart eingerichtet ist, dass das Wechselspannungssignal eine rechteckige Anstiegsflanke hat, vorzugsweise eine Rechteckspannung ist. Die Erfindung betrifft ferner ein Stechelement für ein derartiges Stechgerät sowie ein Stechsystem.

## Beschreibung

Die Erfindung betrifft ein Stechgerät zum Erzeugen einer Einstichwunde zur Gewinnung einer Körperflüssigkeitsprobe eines Menschen oder Tieres, umfassend einen Stechelementantrieb, um ein in das Stechgerät eingesetztes Stechelement für eine Einstich- und Rückführbewegung anzutreiben, eine Wechselspannungsquelle, die dafür eingerichtet ist, ein Wechselspannungssignal an eine in ein eingesetztes Stechelement integrierte Elektrode anzulegen, eine elektrische Messeinrichtung, um ein von dem Wechselspannungssignal hervorgerufenes Antwortsignal zu messen, und eine Auswerteeinrichtung, die im Betrieb durch Auswertung des Antwortsignals ein Auswerteergebnis ermittelt und den Stechelementantrieb unter Berücksichtigung des Auswerteergebnisses steuert.

Die Erfindung betrifft ferner ein Stechsystem umfassend ein derartiges Stechgerät und ein Stechelement für ein derartiges Stechgerät. Die Erfindung betrifft ferner ein Verfahren zur Bestimmung der zur Gewinnung einer Körperflüssigkeitsprobe eines Menschen oder Tieres erforderlichen Einstichtiefe.

Ein stetes Ziel bei der Entwicklung von Stechgeräten zur Gewinnung von Körperflüssigkeitsproben liegt in der Minimierung des mit dem Einstich verbundenen Schmerzes. Von besonderer Bedeutung für das Schmerzempfinden ist dabei die Einstichtiefe, mit der das Stechelement in die Haut eines Patienten eingestochen wird. Idealerweise sollte die Einstichtiefe nur so groß sein, wie es zum Gewinnen einer Probe unbedingt erforderlich ist. Während bei einer unzureichenden Einstichtiefe keine oder nur eine ungenügende Menge Probenflüssigkeit gewonnen wird, führt eine Einstichtiefe, die über das erforderliche Maß hinausgeht, zu unnötigem Schmerz.

Üblicherweise verfügen Stechgeräte nach dem Stand der Technik deshalb über eine Einstelleinrichtung mit der die Einstichtiefe eingestellt werden kann. Optimale Einstellungen werden in der Regel auf der Grundlage von Erfahrungswerten oder durch Ausprobieren ermittelt. Da die optimale Einstichtiefe von der Dicke des Stratum Corneums abhängt, können für einen bestimmten Einstichort eines bestimmten Patienten ermittelte Werte nicht auf andere Einstichorte an anderen Körperteilen oder gar an anderen Patienten übertragen werden. Zur Gewinnung von Körperflüssigkeitsproben werden deshalb häufig unnötig tiefe Einstiche vorgenommen, so dass Patienten Schmerz zugefügt wird, der bei einer optimalen Einstellung der Einstichtiefe vermeidbar wäre.

Da sich die Impedanz des·Stratum Corneums von der Impedanz darunter liegender Hautschichten und insbesondere der Impedanz von Körperflüssigkeit unterscheidet, kann im Prinzip während des Einstichvorgangs durch eine Impedanzmessung festgestellt werden, ob die zum Gewinnen von Körperflüssigkeit erforderliche Einstichtiefe erreicht ist. Bei Stechelementen mit integrierten Elektroden führt eine Benetzung mit Körperflüssigkeit dazu, dass eine Impedanzänderung gemessen werden kann, die darauf schließen lässt, dass das Stechelement mit der zum Gewinnen einer Körperflüssigkeitsprobe erforderliche Einstichtiefe in die Haut eingedrungen ist. Entsprechende Stechgeräte mit Impedanzmesseinrichtungen und Stechelemente mit integrierten Elektroden sind beispielsweise aus der DE 19914485 C2 und der WO 2004/080306 A1 bekannt. Bei diesen Geräten ist eine Impedanzmesseinrichtung an einen Mikroprozessor angeschlossen, der den Stechelementantrieb steuert, so dass das Stechelement zurückgezogen wird, sobald anhand einer Impedanzänderung festgestellt wird, dass die zum Gewinnen einer Körperflüssigkeitsprobe erforderliche Einstichtiefe erreicht ist.

Neben der Optimierung der Einstichtiefe ist zur Schmerzminimierung eine möglichst hohe Einstichgeschwindigkeit erstrebenswert. Je größer jedoch die Geschwindigkeit ist, mit der das Stechelement in die Haut eingestochen wird, desto schneller muss eine Impedanzmessung ausgewertet und durch das Ansteuerung des Stechelementantriebs eine Umkehr der Bewegungsrichtung des Stechelements veranlasst werde. Um eine möglichst schnelle Auswertung der Impedanzmessung zu ermöglichen wird in der WO 2004/080306 A1 die Verwendung einer sinusförmigen Wechselspannung mit einer Frequenz zwischen 10 kHz und 1 MHz bei einer Stromstärke zwischen 1 mA und 10 mA empfohlen. Es hat sich jedoch gezeigt, dass sich auf diese Weise keine ausreichenden Reaktionszeiten erreichen lassen, um Stechvorgänge von wenigen Millisekunden Dauer zu steuern.

Wohl in Anbetracht der Probleme einer raschen Auswertung einer Impedanzmessung wird in der DE 19914485 C2 empfohlen, das Stechelement schrittweise vorzuschieben, bis anhand einer Impedanzänderung eine Benetzung des Stechelements und damit das Erreichen einer ausreichenden Einstichtiefe festgestellt wird. Ein schrittweises Vorschieben des Stechelements führt jedoch dazu, dass der Einstichvorgang länger andauert und dabei insbesondere durch unwillkürliche Bewegungen des Patienten zusätzliche Schmerzen verursacht werden können.

Aufgabe der Erfindung ist es deshalb, einen Weg aufzuzeigen, wie bei einem Einstich schneller eine Benetzung des verwendeten Stechelements mit Körperflüssigkeit festgestellt werden kann.

Diese Aufgabe wird erfindungsgemäß dadurch gelöst, dass die Wechselspannungsquelle derart eingerichtet ist, dass das Wechselspannungssignal eine rechteckige Anstiegsflanke hat, vorzugsweise eine Rechteckspannung ist, und die Anstiegsflanke der Wechselspannung eine Spannungsamplitude von 10 mV bis 200 mV hat.

Überraschenderweise zeigt sich bei Verwendung einer Rechteckspannung deutlicher und schneller eine charakteristische Impedanzänderung bei Benetzung des Stechelements mit Körperflüssigkeit als dies bei Verwendung einer Sinusspannung der Fall ist. Im Rahmen der Erfindung wurde festgestellt, dass bei einem Einstich eine Flanke einer Rechteckspannung ein Antwortsignal auslöst, das durch eine Anstiegsflanke und einen charakteristischen Abfall über einen Zeitraum von wenigen Mikrosekunden gekennzeichnet ist. Bei Verwendung einer Rechteckspannung lässt sich deshalb die Auswertung der im Stand der Technik vorgenommenen Impedanzmessung auf die Auswertung eines kurzen Antwortsignals, vorzugsweise eines Strompulses, reduzieren, das durch eine Flanke der Rechteckspannung ausgelöst wurde. Auf diese Weise kann eine Benetzung der Elektrode eines Stechelements innerhalb von wenigen Mikrosekunden oder sogar Bruchteilen von Mikrosekunden erkannt werden.

Wesentlich für die vorliegende Erfindung ist die steile Anstiegsflanke einer Rechteckspannung. Eine derartige rechteckige Anstiegsflanke ist aber nicht nur bei Rechteckspannungen im engeren Sinne vorhanden, bei denen die Spannung periodisch zwischen genau zwei Spannungswerten wechselt. Für die vorliegende Erfindung ist es unschädlich, wenn die verwendete Spannung beispielsweise von einem ersten Extremwert (z. B. 50 mV) nach einer Zeitspanne D1 auf einen zweiten Wert wechselt (z. B. 0 mV) und von dort nach einer zweiten Zeitspanne D2 auf einen zweiten Extremalwert (z. B. -50 mV) wechselt und von dort nach einer dritten Zeitspanne D3 wieder auf den Zwischenwert (z. B. 0 mV) wechselt. Im Rahmen der Anmeldung sind deshalb Spannungen mit einer

Die Erfindung betrifft ferner ein Stechsystem umfassend ein erfindungsgemäßes Stechgerät und ein auswechselbares Stechelement mit einer integrierten Elektrode. Prinzipiell kann für die integrierte Elektrode jedes beliebige Elektrodenmaterial, beispielsweise Edelstahl oder ein Platinmetall, verwendet werden. Besonders vorteilhaft ist die Verwendung von Platinschwarz als Elektrodenmaterial für die integrierte Elektrode. Platinschwarz kann durch elektrolytische Abscheidung von Platin gewonnen werden und zeichnet sich durch eine sehr poröse und raue Oberflächenstruktur aus. Die Erfindung betrifft deshalb auch ein Stechelement, das eine integrierte Elektrode mit einem Überzug aus Platinschwarz aufweist. Im Rahmen der Erfindung konnte nämlich festgestellt werden, dass bei Verwendung derartiger Stechelemente geringere Übergangswiderstände auftreten, so dass sich die Messsensitivität verbessern lässt.

Die genannte Aufgabe wird ferner gelöst durch ein Verfahren zur Bestimmung der zu Gewinnung einer Körperflüssigkeitsprobe eines Menschen oder Tieres erforderlichen Einstichtiefe eines Stechelements in eine Hautoberfläche, wobei mit einer in das Stechelement integrierten Elektrode eine elektrische Messung durchgeführt wird, um festzustellen, ob das Stechelement mit Körperflüssigkeit benetzt ist, dadurch gekennzeichnet, dass für die Messung eine Rechteckspannung an die Elektrode angelegt wird.

Im Rahmen der Erfindung wurde festgestellt, dass sich bereits mit wesentlich geringeren als den in der WO 2004/080306 A1 empfohlenen Stromstärken zwischen 1 mA . und 10 mA aussagekräftige Messungen durchführen lassen. Bevorzugt sind deshalb die Wechselspannungsquelle und die Elektrode des Stechelements derart aufeinander abgestimmt, dass bei einem Einstich ein Wechselstrom mit einer Stromamplitude von höchstens 500 pA, besonders bevorzugt höchstens 200 pA fließt. Unter der Stromamplitude ist dabei der Spitzenwert der Stromantwort zu verstehen, die durch eine Flanke der Rechteckspannung ausgelöst wird. Bevorzugt hat die verwendete Rechteckspannung eine Spannungsamplitude von 10 mV bis 200 mV, besonders bevorzugt von 10 mV bis 100 mV, insbesondere von 20 mV bis 70 mV. Unter der Spannungsamplitude ist dabei der halbe Wert der bei einer Flanke der Rechteckspannung stattfindenden Spannungsänderung zu verstehen.

Weitere Einzelheiten und Vorteile der Erfindung werden im folgendenen anhand eines Ausführungsbeispiels unter Bezugnahme auf die beigefügten Figuren erläutert. Die darin dargestellten Besonderheiten können einzeln und in Kombination verwendet werden, um bevorzugte Ausgestaltungen zu schaffen. Es zeigen:
- Fig. 1: eine schematische Darstellung eines Stechelements mit integrierter Elektrode beim Eindringen in Haut;
- Fig. 2: eine schematische Darstellung eines erfindungsgemäßen Stechgeräts;
- Fig. 3: ein Beispiel des zeitlichen Verlaufs einer Rechteckspannung;
- Fig. 4: ein Beispiel eines gemessenen Antwortsignals beim Eindringen eines Stechelements in Haut; und
- Fig. 5: ein Beispiel eines Ersatzschaltbildes einer erfindungsgemäß vorgenommenen Messung.

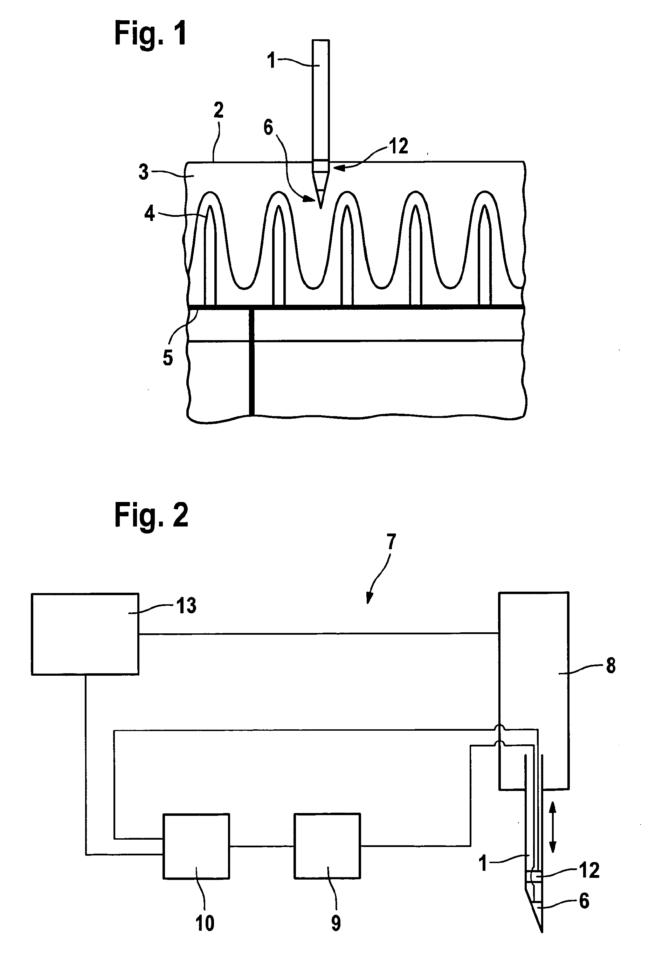

Figur 1 zeigt in einer schematischen Querschnittsdarstellung den Aufbau der Haut und die Verhältnisse bei einem Einstich mit einem Stechelement 1. Die Hautoberfläche wird von dem Stratum Corneum 2 gebildet, unter dem sich eine Kapillarschicht 3 mit Kapillarschlingen 4 befindet. Die Kapillarschlingen 4 werden von blutspeichernden Venolen 5 gespeist.

Während beispielsweise die oberen Hautschichten unterhalb des Stratum Corneums bei Messfrequenzen von weniger als 100 kHz eine elektrische Leitfähigkeit von nur etwa 2 mS/cm haben, haben Körperflüssigkeiten wie beispielsweise Blut, interstitielle Flüssigkeit oder Blutplasma eine mindestens doppelt so große elektrische Leitfähigkeit, so dass durch eine elektrische Messung festgestellt werden kann, ob eine in ein Stechelement 1 integrierte Elektrode 6 mit Körperflüssigkeit benetzt und folglich eine zur Gewinnung einer Körperflüssigkeitsprobe ausreichende Einstichtiefe erreicht ist oder nicht. Die Leitfähigkeit des Stratum Corneums ist wesentlich geringer als die Leitfähigkeit darunter liegender Hautschichten.

Das in Figur 1 dargestellte Stechelement 1 ist Teil eines Stechsystems, das neben auswechselbaren Stechelementen 1 ein Stechgerät umfasst, in das die Stechelemente 1 eingesetzt werden können. Der prinzipielle Aufbau des Stechgeräts mit einem eingesetzten Stechelement 1 ist schematisch in Figur 2 dargestellt. Das Stechgerät 7 umfasst einen Stechelementantrieb 8 um ein in das Stechgerät 7 eingesetztes Stechelement 1 für eine Einstich- und Rückführbewegung anzutreiben, eine Wechselspannungsquelle 9, die dafür eingerichtet ist, ein Wechselspannungssignal an die Elektrode 6 eines eingesetzten Stechelements 1 anzulegen, und eine elektrische Messeinrichtung 10, um ein von dem Wechselspannungssignal hervorgeworfenes Antwortsignal zu messen.

Das Stechgerät 7 kann ferner eine Elektrode, insbesondere eine Ringelektrode, umfassen, die bestimmungsgemäß bei einem Einstich an der Haut anliegt und die Gegenelektrode zu der Elektrode 6 eines Stechelements 1 bildet. Bevorzugt ist das Stechgerät aber zur Verwendung mit Stechelementen 1 ausgebildet, die eine Gegenelektrode 12 zu der Elektrode 6 aufweisen. Ein derartiger bipolarer Aufbau ermöglicht eine bessere Messsensitivität, indem die Messstrecke zwischen der Elektrode 6 und der Gegenelektrode 12 vollständig benetzt werden kann.

Das Stechgerät 7 enthält ferner eine Auswerteeinrichtung 13 in Form eines Mikroprozessors, die im Betrieb durch Auswertung des Antwortsignals ein Auswerteergebnis ermittelt und den Stechelementantrieb 8 unter Berücksichtigung des Auswerteergebnisses steuert. Auf diese Weise kann die Rückführbewegung zum Zurückziehen des Stechelements 1 eingeleitet werden, sobald eine Benetzung der in das Stechelement 1 integrierten Elektrode 6 festgestellt wird und auf diese Weise die Einstichtiefe zur Schmerzminimierung auf das zur Probengewinnung erforderliche Minimum beschränkt werden.

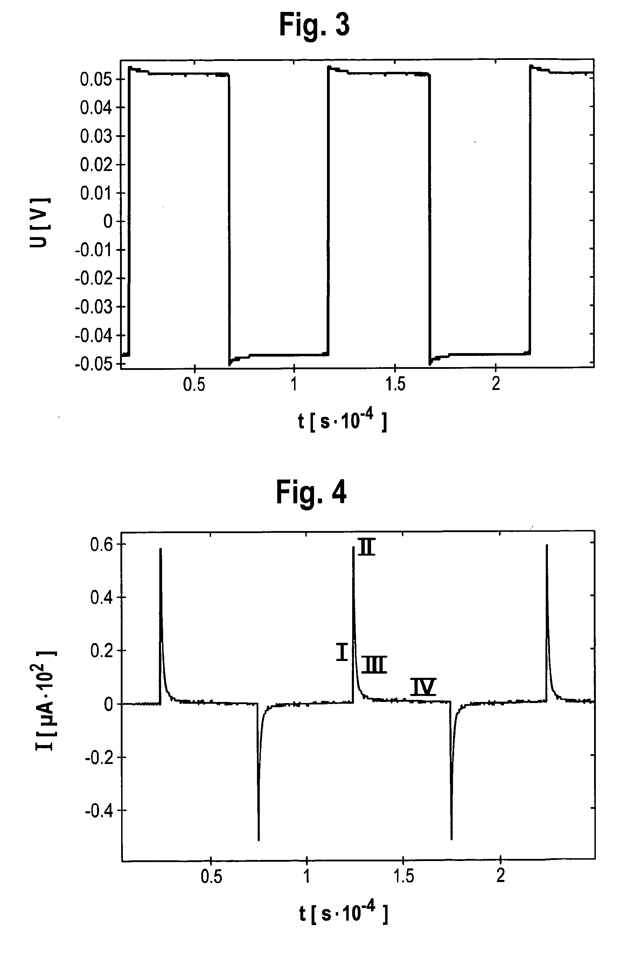

Eine wichtige Besonderheit des dargestellten Stechgeräts 7 besteht darin, dass die Wechselspannungsquelle 9 als Wechselspannungssignal eine Rechteckspannung erzeugt. In Figur 3 ist ein Beispiel des zeitlichen Verlaufs einer geeigneten Rechteckspannung dargestellt. Dabei ist auf der Abszisse die Zeit t in 10⁻⁴ Sekunden und auf der Ordinate die Spannung U in Volt angegeben. Bevorzugt hat die Rechteckspannung eine Spannungsamplitude von weniger als 200 mV, besonders bevorzugt 10 mV bis 100 mV, insbesondere 20 mV bis 60 mV. Die in Figur 2 dargestellte Rechteckspannung hat eine Spannungsamplitude von 50 mV und eine Frequenz von etwa 100 kHz. Die Frequenz der verwendeten Rechteckspannung sollte mindestens 10 kHz betragen. Gut geeignet sind Frequenzen von 10 kHz bis 1 MHz, insbesondere 10 kHz bis 500 kHz. Besonders gut geeignet sind Frequenzen von 50 kHz bis 100 kHz.

Mit dem in Figur 2 dargestellten Aufbau kann das in Figur 3 dargestellte Wechselspannungssignal verwendet werden, um die elektrische Impedanz zwischen den in das Stechelement 1 integrierten Elektrode 6 und 12 zu messen. In Figur 4 ist beispielhaft das Antwortsignal dargestellt, das bei einer solchen Messung von dem Wechselspannungssignal hervorgerufen wird. In Figur 4 ist als Antwortsignal die Stärke des zwischen den Elektroden 6, 12 fließenden elektrischen Stroms I in pA über der Zeit t in 10⁻⁴ s aufgetragen. Selbstverständlich kann als Antwortsignal aber auch die zwischen den beiden Elektroden 6, 12 abfallende Spannung betrachtet werden.

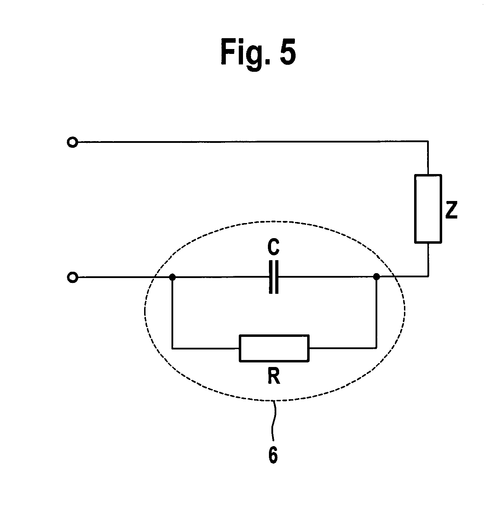

Der Verlauf des dargestellten Antwortsignals lässt sich in vier typische Phasen I, II, III, IV, unterteilen, die im folgenden unter Bezugnahme auf das in Figur 5 dargestellte Ersatzschaltbild erläutert werden. In Figur 5 ist die Elektrode 6 des Stechelements 1 vereinfacht als eine Parallelschaltung einer Kapazität C und eines ohmschen Widerstandes R dargestellt. Gewebe und/oder Körperflüssigkeit zwischen der Elektrode 6 des Stechelements 1 und der Elektrode 12 des Stechgeräts 7 bewirkt die in Figur 5 dargestellte Impedanz Z.

In Figur 4 ist die Phase I durch einen sprunghaften Anstieg des Stromflusses gekennzeichnet. Der Beginn der Phase I fällt mit einer Flanke der Rechteckspannung zusammen. Bei einer idealen Rechteckspannung mit einer unendlich steilen Flanke ist die Dauer der Phase I unendlich kurz. Ideal auftretende Rechteckspannungen gemäß der beispielhaften Darstellung von Figur 3 haben jedoch keine unendlich steile Flanke, so dass sich eine endliche, wenn auch sehr kurze, Zeitdauer der Phase I ergibt.

Die Phase I mündet in die Phase II, in der ein durch eine Flanke der Rechteckspannung ausgelöster Spitzenstrom auftritt. Wie groß der Spitzenstrom ist, hängt gemäß dem in Figur 5 dargestellten Ersatzschaltbild bei Vernachlässigung von ohmschen Streuwiderständen verschiedener Leitungen im Wesentlichen nur von der Impedanz Z des Gewebes und/oder der Körperflüssigkeit zwischen den Elektroden ab. Der in Figur 5 dargestellte Widerstand R, der auf einer Grenzschicht zwischen der Elektrode und umgebenden Gewebe und/oder Körperflüssigkeit beruht, liegt nämlich parallel zu der Kapazität C. Aus diesem Grund lässt sich bereits durch Auswertung des in der Phase II auftretenden Spitzenstroms feststellen, ob die Elektrode des Stechelements 1 von Körperflüssigkeit benetzt ist und folglich eine zur Gewinnung einer Körperflüssigkeitsprobe ausreichende Einstichtiefe erreicht ist.

An die Phase II schließt eine Phase III an, in der sich die von der Elektrode gebildete Kapazität C über den Widerstand C entlädt, so dass es zu einem exponentiellen Abfall der Stromstärke mit der Charakteristik eines RC-Glieds kommt. Der maßgebliche Widerstand R für das Entladen der Kapazität C beruht im Wesentlichen auf der Grenzschicht zwischen der Elektrode und umgebendem Gewebe und/oder Körperflüssigkeit, so dass sich prinzipiell auch durch Auswerten der Phase III feststellen lässt, ob die Elektrode mit Körperflüssigkeit benetzt ist.

Bei dem in Figur 5 dargestellten Antwortsignal schließt an die Phase III eine Phase IV an, in der kein Strom mehr fließt. Die Dauer der Phase IV hängt im Wesentlichen von der Frequenz der Rechteckspannung ab. Bei Verwendung hoher Frequenzen kann die Phase IV verschwinden.

Bevorzugt sind die Elektrode 6 des Stechelements 1 und die Wechselspannungsquelle 9 des Stechgeräts 7 derart aufeinander abgestimmt, dass bei Benetzung des Stechelements 1 mit Körperflüssigkeit der durch die Flanke der Rechteckspannung ausgelöste Spitzenstrom des Antwortsignals innerhalb von 1 µs exponentiell auf weniger als die Hälfte abfällt. Besonders günstig ist es, wenn der Spitzenstrom innerhalb von 1 µs auf weniger als 50 µA, vorzugsweise auf weniger als 30 µA, insbesondere auf weniger als 20 pA, abfällt.

Hat die Elektrode 6 des Stechelements 1 einen Überzug aus Platinschwarz, so ergibt sich ein vorteilhaft kleiner Übergangswiderstand der Elektrode, so dass eine Benetzung leichter festgestellt werden kann.

Bevorzugt hat auch die in das Stechelement 1 integrierte Gegenelektrode 12 einen Überzug aus Platinschwarz.

Um eine Benetzung der Elektrode 6 des Stechelements 1 mit Körperflüssigkeit möglichst frühzeitig und zuverlässig detektieren zu können, ist es vorteilhaft, wenn die Elektrode 6 eine Elektrodenfläche von weniger als 10000 µm², bevorzugt weniger als 6000 µm², besonders bevorzugt weniger als 4000 µm² und insbesondere weniger als 3000 µm², hat. Bevorzugt hat auch die Gegenelektrode 12 eine Elektrodenfläche von weniger als 10000 µm², besonders bevorzugt weniger als 6000 µm², insbesondere weniger als 4000 µm². Kleine Elektrodenflächen haben den Vorteil, Störeinflüsse zu minimieren. Für die in Figur 4 gezeigten Messungen wurde ein Stechelement verwendet, dessen Elektroden 6 und 12 Flächen von etwa 2900 µm² bzw. 2600 µm² haben.

Die beiden Elektroden 6 und 12 sind auf dem Stechelement 1 in einem Abstand von einigen 10 µm angeordnet. Günstig ist ein Elektrodenabstand von nicht mehr als 100 µm, besonderes günstig ein Abstand von 20 µm bis 30 µm.

## Patentansprüche

1. Stechgerät zum Erzeugen einer Einstichwunde zur Gewinnung einer Körperflüssigkeitsprobe eines Menschen oder Tieres, umfassend
einen Stechelementantrieb (8), um ein in das Stechgerät (7) eingesetztes Stechelement (1) für eine Einstich- und Rückführbewegung anzutreiben,
eine Wechselspannungsquelle (9), die dafür eingerichtet ist, ein Wechselspannungssignal an eine in ein eingesetztes Stechelement (1) integrierte Elektrode (6) anzulegen,
eine elektrische Messeinrichtung (10), um ein durch das Anlegen des Wechselspannungssignals an die Elektrode (6) hervorgerufenes Antwortsignal zu messen, und
eine Auswerteeinrichtung (13), die im Betrieb durch Auswertung des Antwortsignals ein Auswerteergebnis ermittelt und den Stechelementantrieb (8) unter Berücksichtigung des Auswerteergebnisses steuert, wobei
die Wechselspannungsquelle (9) derart eingerichtet ist, dass das Wechselspannungssignal eine rechteckige Anstiegsflanke hat
**dadurch gekennzeichnet, dass**
die Anstiegsflanke der Wechselspannung eine Spannungsamplitude von 10 mV bis 200 mV hat.

2. Stechgerät nach Anspruch 1, **dadurch gekennzeichnet, dass** die Wechselspannung einer Rechteckspannung ist.

3. Stechgerät nach einem der vorgehenden Ansprüche, **dadurch gekennzeichnet, dass** die Rechteckspannung eine Spannungsamplitude von 10 mV bis 100 mV, insbesondere 20 mV bis 70 mV, hat.

4. Stechgerät nach einem der vorhergehenden Ansprüche, **dadurch gekennzeichnet, dass** die Rechteckspannung eine Frequenz von 10 kHz bis 1 MHz, vorzugsweise 10 kHz bis 500 kHz, insbesondere 50 kHz bis 100 kHz, hat.

5. Stechgerät nach einem der vorhergehenden Ansprüche, **dadurch gekennzeichnet, dass** es zur Verwendung mit Stechelementen (1) ausgebildet ist, die eine Gegenelektrode (12) zu der Elektrode (6) aufweisen.

6. Stechsystem umfassend ein Stechgerät (7) nach einem der vorhergehenden Ansprüche und ein auswechselbares Stechelement (1) mit einer integrierten Elektrode (6).

7. Stechsystem nach Anspruch 6, **dadurch gekennzeichnet, dass** das auswechselbare Stechelement (1) eine Gegenelektrode (12) zu der Elektrode (6) aufweist.

8. Stechsystem nach Anspruch 6 oder 7, **dadurch gekennzeichnet, dass** die Wechselspannungsquelle (9) und die Elektrode (6) des Stechelements (1) derart aufeinander abgestimmt sind, dass bei einem Einstich ein Wechselstrom mit einer Stromamplitude von höchstens 500µA, vorzugsweise höchstens 200 µA fließt.

9. Stechsystem nach einem der Ansprüche 6 bis 8, **dadurch gekennzeichnet, dass** die Elektrode (6) des Stechelements (1) und die Wechselspannungsquelle (9) derart aufeinander abgestimmt sind, dass bei Benetzung des Stechelements mit Körperflüssigkeit ein durch eine Flanke der Rechteckspannung ausgelöster Spitzenwert des Antwortsignals innerhalb von 1 µs auf weniger als die Hälfte abfällt.

10. Stechelement für ein Stechsystem nach einem der Ansprüche 6 bis 9, **dadurch gekennzeichnet, dass** es eine Elektrode (6) mit einem Überzug aus Platinschwarz aufweist.

11. Stechelement nach Anspruch 10, **dadurch gekennzeichnet, dass** die Elektrode (6) eine Elektrodenfläche von weniger als 10000 µm2, bevorzugt weniger als 6000 µm2, besonders bevorzugt weniger als 4000 µm2, insbesondere weniger als 3000 µm2, hat.

12. Verfahren zur Bestimmung der zur Gewinnung einer Körperflüssigkeitsprobe eines Menschen oder Tieres erforderlichen Einstichtiefe eines Stechelements (1), wobei mit einer in das Stechelement (1) integrierten Elektrode (6) eine elektrische Messung durchgeführt wird, um festzustellen, ob das Stechelement (1) mit Körperflüssigkeit benetzt ist, **dadurch gekennzeichnet, dass** für die Messung eine Rechteckspannung an die Elektrode (6) angelegt wird.
